# EUROPÄISCHE PATENTSCHRIFT

(11) **EP 1 217 986 B1**
(45) Veröffentlichungstag und Bekanntmachung des Hinweises auf die Patenterteilung: **09.04.2003**
(21) Anmeldenummer: 00979426.4
(22) Anmeldetag: 06.10.2000
(51) Int. Cl.: A61K 7/46

(54) **PARFÜM MIT MÖGLICHKEITEN ZUR ÄNDERUNG DER DUFTNOTE**
PERFUME WHOSE FRAGRANCE CAN BE CHANGED
PARFUM OFFRANT DES POSSIBILITES DE MODIFICATION DES FRAGRANCES

(30) Priorität: 08.10.1999 DE 19949418
(43) Veröffentlichungstag der Anmeldung: 03.07.2002
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: DE0003598
(87) Internationale Veröffentlichungsnummer: WO01026612

(56) Entgegenhaltungen:
- EP-A- 0 692 239
- DE-C- 4 100 490
- DATABASE WPI Derwent Publications Ltd., London, GB; AN 1989-275100 XP002163851 & JP 01 201261 A (NIPPON SEIKA), 14. August 1989 (1989-08-14)
- DATABASE WPI Derwent Publications Ltd., London, GB; AN 1980-35635c XP002163852 & JP 55 047611 A (POLA KASEI), 4. April 1980 (1980-04-04)

## Beschreibung

Die Erfindung betrifft ein Parfüm, das wenigstens zwei vom Anwender wählbare Duftnoten enthält.

Parfüme mit unterschiedlichen Duftnoten werden bereits seit Jahrtausenden benutzt. Mit der industrialisierten Herstellung von etherischen ölen und der Synthese von Riechstoffen ist sowohl die Verfügbarkeit als auch die Anwendungsbreite wesentlich verbessert worden. Schwerpunkt in der Parfümerie ist die Kreierung neuer Düfte, die sich mitunter aus über 100 Einzelkomponenten zusammensetzen, sowie die Fixierung der Basisnote, um die Bindung und Haftfestigkeit beim Anwender/bei der Anwenderin zu erhöhen. Da Parfüms ein Massenprodukt geworden sind, geht das Bestreben der Anwender dahin, möglichst unterschiedliche Düfte zur Verfügung zu haben und die Duftnote gegebenenfalls auch schnell wechseln zu können. Das ist bisher nur möglich, indem mehrere unterschiedliche Parfüms vom Anwender mitgeführt und dann wunschgemäß angewendet werden.

Aus der EP-A-692239 ist ein zweiphasiges Parfüm bekannt, das eine wäßrig-alkoholische Phase mit einem Duftstoff enthält sowie eine ein flüchtiges Siliconöl enthaltende Phase. Weiterhin ist aus der US-A-5468496 ein Kosmetikum mit zwei sich schnell trennenden Phasen bekannt, bei dem die wäßrige Phase ein Entmischungsmittel und wenigstens eine der beiden Phasen ein oberflächenaktives Mittel enthält.

JP-A-01 201261 offenbart eine flüssige Parfümzusammensetzung aus wenigstens zwei Schichten, wobei Wasser- und Ölschicht abwechselnd übereinandergeschichtet sein können. Die Wasserschicht enthält Wasser, ein Parfüm und ein oberflächenaktives Mittel.

JP-A-55 047611 offenbart eine Parfümzusammensetzung aus 3 Schichten. Eine Schicht besteht aus einem flüssigen Kohlenwasserstofföl, eine andere Schicht aus Wasser und eine dritte Schicht aus einem Alkohol.

Der Erfindung liegt die Aufgabe zugrunde, flexibel auf die Änderungswünsche des Anwenders einzugehen und ein Parfüm mit Möglichkeiten zur Änderung der Duftnote bereitzustellen.

Erfindungsgemäß besteht das Parfüm aus wenigstens zwei deutlich voneinander getrennten, klaren Schichten, von denen die erste Schicht eine ölphase ist, und die zweite Schicht eine wäßrige, wäßrig-alkoholische oder ein Isostearat-Derivat umfassende Phase ist, mit der Maßgabe, daß wenn die ölphase ein Siliconöl enthält, die zweite Schicht eine durch ein Isostearat-Derivat gebildete Phase ist, wobei die zweite Schicht wenigstens einen wasserlöslichen Duft umfaßt und die erste Schicht wenigstens einen von dem wasserlöslichen Duft verschiedenen öllöslichen Duft umfaßt, und das Gewichtsverhältnis zwischen wäßriger bzw. Isostearatphase und ölphase im Bereich von 25:75 bis 75:25 liegt.

Unter dem Begriff "wasserlöslicher Duft" wird im Rahmen der vorliegenden Erfindung ein aus einer oder mehreren Duftkomponenten bestehender Gesamtduft verstanden, der in Wasser oder gegebenenfalls Wasser/Alkohol in Form einer klaren Lösung löslich ist.

Unter dem Begriff "öllöslicher Duft" wird im Rahmen der vorliegenden Erfindung ein aus einer oder mehreren Duftkomponenten bestehender Gesamtduft verstanden, der in einem kosmetischen öl in Form einer klaren Lösung löslich ist.

Unter dem Begriff "Parfüm" wird im Rahmen der vorliegenden Erfindung die Gesamtkomposition aus wenigstens zwei Phasen verstanden, worin die Konzentration der Düfte im Bereich von 0,2 bis 40 Gew-% insgesamt liegen kann. Es werden daher auch insbesondere geringere Konzentrationen unter 10 Gew-%, bezogen auf das Gesamtgewicht der Komposition, hier als Parfüm bezeichnet.

Die Ölphase besteht vorteilhaft aus einem niedrigviskosen Öl mit Viskositäten von 100 bis etwa 3000 Pa·s, wobei das öl besonders bevorzugt eine Viskosität ähnlich wie Wasser hat. Die Viskosität wird dabei nach der Brookfield-Methode mit einer Spindel # Nr.1 bei 25 °C gemessen.

Das öl wird vorteilhaft aus der Gruppe ausgewählt die aus Fettsäureestern, Siliconölen, pflanzlichen ölen und Gemischen davon besteht. Bevorzugt sind Hexyl Laurate (Cetiol A), Cetyl Alcohol, Jojobaöl, Silicone, Cetearyl Alcohol, Dicapryl Ether, Cetearyl Isononanoate, Palm Oil und Octyl Salicylate.

Wenn das öl, das die erste Schicht bildet, ein Siliconöl ist, wie z.B. Dimethicone oder Cyclomethicone, wird die zweite Schicht von einem Isostearat-Derivat gebildet. Unter dem Begriff "Isostearat-Derivat" werden kosmetisch annehmbare Isostearate verstanden, wie Propylenglycol-diisostearat, Octylisostearat, Isostearylisostearat, Triisostearin, Isostearinsäure, Trimethylolpropan-triisostearat und Pentaerythrityl-tetraisostearat. Besonders bevorzugt sind Trimethylolpropan-triisostearat und Pentaerythrityl-tetraisostearat. Der Einsatz der Isostearat-Derivate ist besonders duftkompatibel, irritationsfrei und für die Phasenmischung vorteilhaft.
Bevorzugte Kombinationen von Phasen sind eine wäßrige Phase und ein wasserlöslicher Duft mit einer Ölphase aus z.B. einem Fettsäuretriglyderid einer mittelkettigen Fettsäure und einem öllöslichen Duft; oder eine wäßrig-alkoholische Phase und z.B. ein dreiwertiger Alkohol wie Propylenglycol und ein wasserlöslicher Duft mit einer ölphase aus z.B. einem Isostearat-Derivat und einem öllöslichen Duft; oder eine Siliconölphase und ein öllöslicher Duft mit einer Phase aus einem Isostearat-Derivat, wie Trimethylolpropan-triisostearat, und einem wasserlöslichen Duft.

Der wasserlösliche Duft wird vorzugsweise ausgewählt unter Orange Oil, Rose Oil, Citronella Oil, Chamomille Oil, Lemon Oil, Lavendel Oil und Gemischen davon und wird in Form handelsüblicher Wässer, z.B. Rosenwasser, Pfefferminzwasser usw. eingesetzt.

Der öllösliche Duft wird vorzugsweise ausgewählt unter Ambre, Anekole C₁₀H₁₂O, Angelique Rook Oil, Artemisia Oil, Basil Oil, Bay Oil, Benzaldehyde C₇H₆O, Bergamot Oil, Benzyl Acetate C₉H₁₀O₂, Camphre C₁₀H₁₆O, Calmusoil, Carotte Oil, Couramine C₉H₆O₂, Cypren Oil, Dihydromyrcenol C₁₀H₂₀O, Jasmin, Mimosa Muscone C₁₆H₃₀O, Narcissus, Sanddorn wood Oil, Vanille Oil, Citrone Oil und Gemischen davon. Die Erfindung ist nicht auf diese Düfte eingeschränkt.

Die Düfte werden vorzugsweise in ihrer Kombination miteinander so ausgewählt, daß bei Vermischen der beiden Phasen ein von den beiden anderen Düften unterschiedlicher Mischduft entsteht, der als dritter Duft dem erfindungsgemäßen Parfüm zuzuordnen ist. Die genannten bevorzugten Parfümöle sind dafür besonders geeignet.

Durch die unterschiedlichen Düfte in den wenigstens zwei Schichten des Parfüms hat der Anwender die Möglichkeit, mit Hilfe einfacher Mittel, wie Pipetten oder Doppelpumpköpfen auf einem Sprühbehälter, aus der jeweiligen Schicht das entsprechend gewünschte Parfüm zu entnehmen. Weiterhin besteht die Möglichkeit, ein drittes Parfüm zu erzeugen, indem die beiden Phasen miteinander vermischt werden und danach der neu entstandene Duft aus der temporären Mischphase entnommen wird. Dabei wird die Auswahl der einzelnen Düfte in der wäßrigen Phase und in der Ölphase entscheidend sein für den Mischduft, dieser kann jedoch von einem Fachmann auf diesem Gebiet ohne Schwierigkeiten festgelegt werden. Diese Verwendung ist ein weiterer Gegenstand der Erfindung.

Um die Trennung von wäßriger Phase und Ölphase nach einem Mischvorgang zu verlangsamen und damit den Zeitraum für die Entnahme des Mischduftes zu verlängern, ist es möglich, entsprechende öle z.B. Triglyceride in gewünschten Anteilen in der ölphase einzusetzen.

Nach einem Mischvorgang und Wiederherstellung der Phasen liegen auch die Düfte wieder in den jeweiligen Phasen vor und können getrennt mit dem Träger der jeweiligen Phase entnommen werden.

Die wäßrige Phase kann bis zu 30 Gew-% eines einwertigen Alkohols enthalten, insbesondere 1 bis 10 % Gew-%, um die Trocknung des Duftes auf der behandelten Oberfläche zu beschleunigen. Als Alkohol kommen hierbei insbesondere C₃-C₅-Alkohole in Frage, wie z.B. Propanol oder Isopropanol. Die wäßrige Phase kann auch mehrwertige Alkohole wie Propylenglycol enthalten in Konzentrationen von 1 bis 10 Gew-%, jeweils bezogen auf das Gesamtgewicht der Zusammensetzung.

Die Düfte in der ölphase können in einem Anteil von 0,1 bis 20 % Gew-% enthalten sein, bezogen auf das Gewicht der jeweiligen Phase, vorzugsweise 1 bis 15 Gew-%, insbesondere 3 bis 15 Gew-%. Eine weitere Ausführungsform der Erfindung kann Düfte im Bereich von 8 bis 20 Gew-% enthalten, ebenfalls bezogen auf das Gewicht der Phase.

In der wäßrigen Phase können die Düfte in einem Anteil von 0,1 bis 15 Gew-% enthalten sein, vorzugsweise 1 bis 10 Gew-%.

In beiden Phasen können zusätzlich kosmetische Hilfs- und Wirkstoffen enthalten sein, wie z.B UV-Filter. Das Vorhandensein von oberflächenaktiven und/oder Entmischungsmitteln wird dabei ausgeschlossen.

Eine weitere Ausführungsform der Erfindung besteht darin, daß mehr als zwei Schichten, z.B. drei Schichten mit unterschiedlichen Düften bereitgestellt werden. Dabei besteht eine Schicht aus einer reinen wäßrigen Phase, eine zweite Schicht aus einer ölphase (z.B. ein Fettsäuretriglycerid, ein Pflanzenöl oder ein Isostearat-Derivat) und eine dritte Schicht aus einer speziellen leichten Siliconölphase, z.B. Cyclomethicone. Letztere setzt sich ebenfalls als deutlich getrennte Schicht ab. Aus jeder Phase kann der Duft einzeln entnommen werden, und ein vierter Gesamtduft ergibt sich durch Schütteln aller Phasen und Entnahme des Duftes unmittelbar nach dem Schütteln. Zu diesem Zeitpunkt liegt für kurze Zeit ein emulsionsartiges Gemisch vor, in dem auch die verschiedenen Düfte einen Mischduft bilden.

Das Parfüm kann bei einem zweiphasigen Ausführungsform in einem Sprühbehälter mit Doppelpumpkopf enthalten sein, bei dem ein Ansaugröhrchen des Pumpkopfes in die ölschicht und in die wäßrige Schicht eintaucht, d.h. bis in die unter der ölschicht liegende wäßrige Schicht reicht, und das andere Ansaugröhrchen allein in die ölschicht eintaucht. Damit ist gewährleistet, daß bei Betätigung des entsprechenden Pumpkopfes der gewünschte Duft vom Pumpkopf gefördert und an der Sprühdüse dann versprüht wird.

In ähnlicher Weise kann auch mittels einer Pipette in Abhängigkeit von der Eintauchtiefe die jeweilig gewünschte Duftnote zusammen mit dem Träger der Phase, z.B. Wasser, entnommen werden.

Auf diese Weise können auch unterschiedliche Düfte für Partner (z.B. Dame/Herr) oder Düfte für verschiedene Tageszeiten in einem Behälter angeboten werden. Ein weiterer Vorteil der Erfindung besteht darin, daß Hautirritationen vermieden werden können, die für viele Anwender mit den hohen Alkoholkonzentrationen von bis zu 80 % bei üblichen Parfümen verbunden sind.

Durch Verschiebung der Phasenträger Wasser oder öl zu den Extremwerten können unterschiedliche Anwendungsformen bereitgestellt werden. So wird beispielsweise mit c. 65-70 Gewichtsanteilen ölphase und dem Rest Wasserphase ein Parfüm-Bodyspray und mit ca. 65-70 Gewichtsanteilen Wasserphase und dem Rest ölphase ein Haarspray herstellbar.

Zusätzlich können in der jeweiligen Phase auch solche Sonnenschutzfilter enthalten sein, die sich entsprechend klar darin lösen. Auch weitere Begleitstoffe für kosmetische Anwendungen können hinzugesetzt werden, soweit eine klare Lösung und eine stabile Phase beibehalten wird.

Die Erfindung soll nachstehend durch Beispiele näher erläutert werden. Alle Prozentangaben sind Gewichtsprozente, wenn nichts anderes angegeben ist.

### Beispiel 1

| **Wäßrige Phase** | |
|---|---|
| Dest. Wasser | q.s. ad 100 |
| Rose Oil (wasserlöslich) | 10 |

| **ölphase** | |
|---|---|
| Jojoba Oil light | q.s. ad 100 |
| cypren Oil (30%) und Jasmin Oil (70%) | 10 |

Die Phasen werden einzeln durch Mischen ihrer jeweiligen Bestandteile hergestellt. Anschließend werden 60 Gewichtsteile der ölphase und 40 Gewichtsteile der Wasserphase nacheinander in einen Parfümbehälter, wie einen Spraybehälter, eingefüllt, der mit einem Doppelpumpkopf und damit verbundenen und entsprechend den ausgebildeten Schichten unterschiedlich langen Ansaugröhrchen versehen ist. Durch Einzelbetätigung der beiden Pumpköpfe kann die jeweilige Phase zerstäubt werden. Nach kräftigem Schütteln des Behälters und Betätigung eines der beiden Pumpköpfe kann ein Mischduft zerstäubt werden.

### Beispiel 2

| **Wäßrige Phase** | |
|---|---|
| Dest. Wasser | 87 |
| Citronella Oil (wasserlöslich) | 10 |
| Benzophenone-4 | 3 |
| **ölphase** | |
| Cetiol Hexyl Laurate | 82,5 |
| Angelique Rooke Oil | 10 |
| Octyl Salicylate | 7,5 |

| **Siliconphase** | |
|---|---|
| Cyclomethicone | 97 |
| Mimosa Muscone | 3 |

Die drei Phasen wurden einzeln vorgemischt und anschließend in folgenden Anteilen in einen Parfümbehälter gegeben: wäßrige Phase 36%, ölphase 30%, Siliconphase 34%. Die Entnahme aus dem Parfümbehälter erfolgt mit einer Pipette aus der gewünschten Schicht oder dem nach Schütteln entstandenen Gemisch, das sich nach einer gewissen Zeit wieder in die einzelnen Schichten auftrennt.

### Beispiel 3

| | |
|---|---|
| Trimethylolpropan-triisostearat | 47 |
| Rosenwasser | 3 |
| Propylenglycol | 3 |
| Wasser | 44 |
| Citrone Oil | 3 |

Es wird wie im Beispiel 1 gemischt, und die beiden Phasen werden im Verhältnis 50:50 in einen Parfümbehälter eingebracht. Durch Vermischen beider Phasen im Behälter wird für eine kurze Zeit ein neuer Mischduft gebildet.

### Beispiel 4

| | |
|---|---|
| Cyclomethicone | 48 |
| Vanille Oil | 2 |
| Trimethylolpropan-triisostearat | 48 |
| Rosenwasser | 2 |

Es wird wie im Beispiel 3 gearbeitet.

## Patentansprüche

1. Parfüm mit Möglichkeiten zur Änderung der Duftnote, **dadurch gekennzeichnet, daß** das Parfüm wenigstens zwei deutlich voneinander getrennte, klare Schichten aufweist, von denen die erste Schicht eine ölphase ist, und die zweite Schicht eine wäßrige, wäßrig-alkoholische oder ein Isostearat-Derivat umfassende Phase ist, mit der Maßgabe, daß wenn die ölphase ein Siliconöl enthält, die zweite Schicht eine durch ein Isostearat-Derivat gebildete Phase ist, wobei die zweite Schicht wenigstens einen wasserlöslichen Duft umfaßt und die erste Schicht wenigstens einen von dem wasserlöslichen Duft verschiedenen öllöslichen Duft umfaßt, und das Gewichtsverhältnis zwischen wäßriger oder Isostearatphase und Ölphase im Bereich von 25:75 bis 75:25 liegt, und wobei beide Schichten kein oberflächenaktives Mittel enthalten.

2. Parfüm nach Anspruch 1, **dadurch gekennzeichnet, daß** die ölphase niedrigviskose Öle mit Viskositäten von 100 bis 3000 Pa·s bei 25°C enthält.

3. Parfüm nach Anspruch 1, **dadurch gekennzeichnet, daß** die Öle ausgewählt sind unter Fettsäureestern, Siliconölen, pflanzlichen ölen und Gemischen davon.

4. Parfüm nach Anspruch 1, **dadurch gekennzeichnet, daß** der wasserlösliche Duft ausgewählt ist unter Orange Oil, Rose Oil, Citronella Oil, Chamomille Oil, Lemon Oil, Lavendel Oil, jeweils in einer wasserlöslichen Form.

5. Parfüm nach Anspruch 1, **dadurch gekennzeichnet, daß** der öllösliche Duft ausgewählt ist unter Ambre, Anekole C₁₀H₁₂O, Angelique Rook Oil, Artemisia Oil, Basil Oil, Bay Oil, Benzaldehyde C₇H₆O, Bergamot Oil, Benzyl Acetate C₉H₁₀O₂, Camphre C₁₀H₁₆O, Calmusoil, Carotte Oil, Couramine C₉H₆O₂, Cypren Oil, Dihydromyrcenol C₁₀H₂₀O, Jasmin, Mimosa Muscone C₁₆H₃₀O, Narcissus, Sanddorn wood Oil und Gemischen davon.

6. Parfüm nach Anspruch 1, **dadurch gekennzeichnet, daß** ein Gemisch von wasserlöslichem Duft und öllöslichem Duft, das beim Vermischen von wäßriger Phase und ölphase vorliegt, einen von den beiden Düften unterschiedlichen dritten Duft aufweist.

7. Parfüm nach Anspruch 1, **dadurch gekennzeichnet, daß** die wäßrige Phase bis zu 30 Gew-% eines einwertigen Alkohols enthält.

8. Parfüm nach Anspruch 7, **dadurch gekennzeichnet, daß** der einwertige Alkohol ein C₃-C₅-Alkohol ist.

9. Parfüm nach Anspruch 1, **dadurch gekennzeichnet, daß** die Düfte in der jeweiligen Phase in einem Anteil von 0,1 bis 20 Gew-% enthalten sind, bezogen auf das Gewicht der jeweiligen Phase.

10. Parfüm nach Anspruch 1, **dadurch gekennzeichnet, daß** das Parfüm in einem Sprühbehälter mit Doppelpumpkopf vorliegt, bei dem ein Ansaugröhrchen in die ölschicht und in die wäßrige Schicht eintaucht und das andere Ansaugröhrchen allein in die ölschicht eintaucht.

11. Parfüm nach Anspruch 1, **dadurch gekennzeichnet, daß** die erste Schicht ein Isostearat-derivat enthält, und die zweite Schicht eine wäßrige Phase darstellt.

12. Parfüm nach Anspruch 1, **dadurch gekennzeichnet, daß** die erste Schicht ein Fettsäuretriglycerid enthält, und die zweite Schicht eine wäßrig-alkoholische Phase mit einem einwertigen C₃-C₅-Alkohol darstellt.

13. Verwendung eines Parfüms nach Anspruch 1, **dadurch gekennzeichnet, daß** aus einer Packungseinheit mittels einer Entnahmevorrichtung aus der jeweiligen Schicht oder aus dem durch Schütteln erzeugten temporären Gemisch der jeweilige Duft mit der entsprechenden Trägerphase entnommen wird.

## Claims

1. A perfume whose fragrance can be changed, which comprises at least two clear layers which are distinctly separated from one another of which the first layer is an oil phase and the second layer is an aqueous, aqueous-alcoholic or isostearate derivative phase wherein, if the oil phase contains a silicone oil, the second layer is a phase formed by an isostearate derivative and wherein the second layer comprises at least one water-soluble fragrance and the first layer comprises at least one oil-soluble fragrance other than the water-soluble fragrance and wherein the ratio of the aqueous or isostearate phase's weight to that of the oil phase is in the range of 25:75 to 75:25.

2. A perfume according to Claim 1 wherein the oil phase contains low-viscosity oils whose viscosity is between 100 and 3,000 Pa·s at 20 °C.

3. A perfume according to Claim 1 wherein the oils are selected from among fatty acid esters, silicone oils, vegetable oils and mixtures thereof.

4. A perfume according to Claim 1 wherein the water-soluble fragrance is selected from among Orange Oil, Rose Oil, Citronella Oil, Chamomile Oil, Lemon Oil, Lavender Oil, each of them in a water-soluble form.

5. A perfume according to Claim 1 wherein the oil-soluble fragrance is selected from among of Ambergris, Anethole C₁₀H₁₂O, Angelique Root Oil, Artemisia Oil, Basil Oil, Bay Oil, Benzaldehyde C₇H₆O, Bergamot Oil, Benzyl Acetate C₉H₁₀O₂, Camphor C₁₀H₁₆O, Calamus Oil, Carrot Oil, Coumarine C₉H₆O₂, Cypress Oil, Dihydromyrcenol C₁₀H₂₀O, Jasmine, Mimosa Muscone C₁₆H₃₀O, Narcissus, Sandalwood Oil and mixtures thereof.

6. A perfume according to Claim 1 wherein a mixture of the water-soluble fragrance and the oil-soluble fragrance which is present after mixing the aqueous phase and the oil phase has a third fragrance other than the two first fragrances.

7. A perfume according to Claim 1 wherein the aqueous phase contains up to 30 % by weight of a monovalent alcohol.

8. A perfume according to Claim 7 wherein the monovalent alcohol is a C₃-C₅ alcohol.

9. A perfume according to Claim 1 wherein the fragrance content of the respective phase is in the range of 0.1 to 20 % by weight relative to the weight of the respective phase.

10. A perfume according to Claim 1 wherein the perfume is present in a spray container with dual pump head in which one suction tube is immersed in the oil layer and the aqueous layer and the other suction tube is immersed in the oil layer only.

11. A perfume according to Claim 1 wherein the first layer contains an isostearate derivative and the second layer is an aqueous phase.

12. A perfume according to Claim 1 wherein the first layer contains a fatty acid triglyceride and the second layer is an aqueous-alcoholic phase containing a monovalent C₃-C₅ alcohol.

13. Use of a perfume according to Claim 1 wherein the respective fragrance together with its carrier phase is drawn from a packing unit, from the respective layer or from the temporary mixture produced by shaking, by means of a drawing device.

## Revendications

1. Parfum offrant des possibilités de modification des fragrances **caractérisé en ce que** le parfum présente au moins deux couches transparentes nettement séparées l'une de l'autre, la première couche étant une phase huileuse et la seconde couche étant une phase aqueuse, une phase aqueuse-alcoolique ou une phase contenant un dérivé d'isostéarate, à la condition que lorsque la phase huileuse contient une huile de silicone, la seconde couche est une phase formée par un dérivé d'isostéarate, la seconde couche contenant au moins une senteur hydrosoluble et la première couche contenant au moins une senteur soluble dans l'huile différente de la senteur hydrosoluble, le rapport en poids entre la phase aqueuse ou d'isostéarate et la phase huileuse étant compris entre 25:75 et 75:25, les deux couches ne contenant pas d'agent de surface actif.

2. Parfum selon la revendication 1 **caractérisé en ce que** la phase huileuse contient des huiles faiblement visqueuses ayant des viscosités comprises entre 100 et 3 000 pA.s à 25 °C.

3. Parfum selon la revendication 1 **caractérisé en ce que** les huiles sont choisies parmi les esters d'acide gras, les huiles de silicone, les huiles végétales et leurs mélanges.

4. Parfum selon la revendication 1 **caractérisé en ce que** la senteur hydrosoluble est choisie parmi l'huile d'orange, l'huile de rose, l'huile de citronnelle, l'huile de camomille, l'huile de citron, l'huile de lavande, chacune dans une forme hydrosoluble

5. Parfum selon la revendication 1 **caractérisé en ce que** la senteur soluble dans l'huile est choisie parmi l'ambre, l'anécole C₁₀H₁₂O, l'huile de la racine d'angélique, l'huile d'armoise, l'essence de basilic, l'huile de laurier, le benzaldéhyde C₇H₆O, l'huile de bergamote, le benzyle acétate C₉H₁₀O₂, le camphre C₁₀H₁₆O, l'huile de camus, l'huile de carotte, la couramine C₉H₆O₂, l'huile de cyprène, le dihydromyrcenol C₁₀H₂₀O, le jasmin, la muscone de mimosa C₁₆H₃₀O, le narcisse, l'huile d'argousier et leurs mélanges.

6. Parfum selon la revendication 1 **caractérisé en ce qu'**un mélange de la senteur hydrosoluble et de la senteur soluble dans l'huile obtenu par mélange de la phase aqueuse et de la phase huileuse, entraîne une troisième senteur différente des deux senteurs.

7. Parfum selon la revendication 1 **caractérisé en ce que** la phase aqueuse comprend jusqu'à 30 % en poids d'un alcool monovalent.

8. Parfum selon la revendication 7 **caractérisé en ce que** l'alcool monovalent est un alcool C₃-C₅.

9. Parfum selon la revendication 1 **caractérisé en ce que** les senteurs dans chaque phase sont comprises en une proportion de 0,1 à 20 % en poids du poids de chaque phase.

10. Parfum selon la revendication 1 **caractérisé en ce que** le parfum est présenté dans un récipient pour vaporisation doté d'une tête de pompe double, un tube d'admission étant plongé dans la couche huileuse et dans la couche aqueuse et l'autre tube d'admission étant plongé seul dans la couche huileuse.

11. Parfum selon la revendication 1 **caractérisé en ce que** la première couche contient un dérivé d'isostéarate et la deuxième couche est une phase aqueuse.

12. Parfum selon la revendication 1 **caractérisé en ce que** la première couche contient un triglycéride d'acide gras et **en ce que** la deuxième couche est une phase aqueuse-alcoolique avec un alcool monovalent C₃-C₅.

13. Utilisation d'un parfum selon la revendication 1 **caractérisée en ce qu'**on prélève d'un seul récipient à l'aide d'un dispositif de prélèvement d'une couche particulière ou du mélange temporaire obtenu par agitation la senteur particulière avec la phase de porteur correspondante.
